# EUROPEAN PATENT SPECIFICATION

(11) **EP 1 437 407 B1**
(45) Date of publication and mention of the grant of the patent: **24.12.2008**
(21) Application number: 02730752.9
(22) Date of filing: 29.05.2002
(51) Int. Cl.: C12N 15/10, C12Q 1/68

(54) **APPARATUS FOR COLLECTING NUCLEIC ACID AND METHOD OF COLLECTING NUCLEIC ACID**
VORRICHTUNG UND VERFAHREN ZUM SAMMELN VON NUKLEINSÄURE
DISPOSITIF ET PROCEDE DE COLLECTE D'ACIDES NUCLEIQUES

(30) Priority: 28.09.2001 WO PCT/JP01/08586
(43) Date of publication of application: 14.07.2004
(73) Proprietor: Hitachi, Ltd., Chiyoda-ku, Tokyo 100-8010 (JP)
(72) Inventor: SAKURAI, Toshinari, c/o Instruments Hitachi Ltd, Hitachinaka-shi, Ibaraki 312-8504 (JP); YOKOBAYASHI, Toshiaki, c/o Instruments Hitachi Ltd, Hitachinaka-shi, Ibaraki 312-8504 (JP)
(74) Representative: Strehl Schübel-Hopf & Partner
(86) International application number: PCT/JP2002/005209
(87) International publication number: WO 2003/031619

(56) References cited:
- JP-A- 11 266 864
- JP-A- 2000 166 556
- US-B1- 6 274 726
- THE JAPANESE BIOCHEMICAL SOCIETY, 26 February 1990, TOKYO KAGAKU DOJIN article 'Shin-seikagaku jikken kouza 1, tanpakushitsu I; bunri seisei seishitsu', pages 161 - 169, XP002963708

## Description

### Technical Field

The present invention relates mainly to a method for recovery of nucleic acids from a solution by capture with a solid phase, a method for production of a nucleic acid-containing solution, and a method for determination of the concentration of nucleic acids in a nucleic acid-containing solution.

### Background Art

Recent advances in molecular biology led to the development of new technologies relating to genes, which made it possible to separate and identify many disease-causing genes. This has motivated those in the field of medicine to adopt the technique of molecular biology for diagnosis and examination, which makes easier the old-fashioned diagnoses involving great difficulties and largely reduces the number of days required for examination.

The above-mentioned advancement owes greatly to the polymerase chain reaction (PCR) to amplify nucleic acids, which has recently been put to practice. The PCR method permits nucleic acids in a mixed solution to be amplified according to the sequence specificity. It is used, for example, to indirectly prove the presence of a very small number of viruses in a serum by amplifying the virus-derived nucleic acids and detecting the amplified nucleic acids. Unfortunately, this PCR method poses several problems when it is used for routine clinical examination. Most important of them is the one involved in pretreatment to be carried out before nucleic acids are extracted from a sample of organism. It has been pointed out that it is important to extract high-purity nucleic acids in the purifying step in order to carry out the PCR method accurately after pretreatment. (Ohshima et al., JJCLA, 22(2), 145-150 (1997)) In other words, the purifying step should be so accomplished as to separate pure nucleic acids containing a minimum of impurities when nucleic acids are extracted from organisms by pretreatment. Several techniques have been proposed as follows for purification of nucleic acids.

Japanese Patent Laid-open No. 11-266864 discloses a method of automatically extracting nucleic acids, with the help of a nucleic acid-capturing tip holding a silica-containing solid phase therein. This method consists of the following steps. First, a nozzle tip is attached to a movable nozzle which causes a sample solution to be aspirated and discharged. An agent to promote the binding of nucleic acids to the solid phase is aspirated from a bottle and then a sample solution containing nucleic acids is aspirated from a sample container. The mixed solution is discharged into a reaction vessel. The nozzle tip to capture nucleic acids is discarded and replaced by a new one. The mixed solution is aspirated into the nozzle tip from the reaction vessel, so that nucleic acids in the mixed solution bind to the solid phase in the nozzle tip. The solution in the nozzle tip is discharged. The nozzle tip is caused to aspirate a cleaning solution from a container for cleaning and then discharge it, so as to clean the solid phase to which nucleic acids have: bound and the inside of the nozzle tip. Finally, an eluent is aspirated into the nozzle tip and the eluate (which contains nucleic acids released from the solid phase) is discharged into a container for a purified product. In this way it is possible to obtain purified nucleic acids from a sample solution containing nucleic acids.

The foregoing method (disclosed in Japanese Patent Laid-open No. 11-266864) easily automates the process for purification of nucleic acids owing to the nucleic acid-capturing tip holding a solid phase therein which is removably attached to a movable nozzle to aspirate and discharge solutions. Flowing the mixed solution in two directions brings about efficient contact between the mixed solution and the solid phase.

In addition, there is disclosed another method of purifying nucleic acids in Patent Laid-open (PCT) No. 515066/2000. This method employs a housing which holds therein a porous polymer matrix of three-dimensional structure containing adsorbing particles. According to the disclosure, the housing holds therein a silica-filled membrane matrix. This housing is attached to the tip of a pipet. The pipet is caused to aspirate a solution of DNA which has been equilibrated with GuHCl or NaI, so that DNA binds to the silica-filled membrane matrix held in the housing. Finally, the thus captured DNA is cleaned and eluted.

Moreover, Japanese Patent Laid-open No. 2001-74756 discloses an improved method for automatically extracting nucleic acids with the help of a nucleic acid-capturing tip holding therein a silica-containing solid phase. The improvement consists in measuring the pressure in the tip, thereby accurately judging how far the solid phase has deteriorated.

U.S. Patent 6,175,409 discloses a new method of using high-performance liquid chromatography to analyze polymeric samples by application to the mobile phase. According to the disclosure, a solution containing polymeric samples is caused to flow in one direction relative to the mobile phase at a certain flow rate. The disclosure is concerned with high-performance liquid chromatography in which the solution flows in one direction relative to the mobile phase, but is not concerned with the apparatus equipped with a nucleic acid-capturing tip.

US 6 274 726 B1 discloses a technology for recovering nucleic acids from a solution by aspirating the solution into a container having a phase capable of capturing the nucleic acids. The aspirated nucleic acids are bound to the phase and then liberated and discharged from the container. The hydrostatic pressure in the container is variable to control the binding of the nucleic acids to the solid phase.

Apparatus for recovery of nucleic acids from a sample solution are disclosed also in JP-2000-166 556 A and US 6 235 471 B1.

### Summary of the Invention

It is an object of the invention to provide a method for improved recovery of nucleic acids from a solution. Also, embodiments of the invention shall be able to produce and to determine the concentration of nucleic acids in a sample.

This object is solved by the method of claim 1. The dependent claims relate to preferred embodiments of the invention.

The gist of the present invention resides mainly in a method for recovery of nucleic acids by using a nucleic acid-capturing container (such as a tip) holding therein a solid phase capable of capturing nucleic acids. According to the present invention, recovery of nucleic acids is accomplished by aspirating and discharging a solution containing nucleic acids, so that nucleic acids are captured on the solid phase and then the captured nucleic acids are released from the solid phase. The yield of recovery is improved by controlling the flow rate at which the sample solution is aspirated or discharged or by controlling the internal pressure of the container, so that adequate contacts take place between the sample solution and the solid phase.

### Brief Description of the Several Views of Drawings

Fig. 1 is a side view showing a nucleic acid-capturing tip holding therein a solid phase.
Fig. 2 is a plan view showing a work bench of the nucleic acid-purifying apparatus.
Fig. 3 is a perspective view showing important parts of the nucleic acid-purifying apparatus.
Fig. 4 is a diagram of important parts illustrating how to attach a dispensing tip to a dispensing nozzle.
Fig. 5 is a plan view of important parts illustrating a nucleic acid-capturing tip 1 attached to a movable nozzle to aspirate and discharge solutions.
Fig. 6 is a schematic diagram illustrating how to remove a dispensing tip from a dispensing nozzle by using a tip remover.
Fig. 7 is a block diagram showing the electric system of the nucleic acid-purifying apparatus.
Fig. 8 is a graphical representation showing the relationship between the amount of nucleic acids contained in the mixed solution before aspiration and the amount of nucleic acids contained in the mixed solution after discharging, in the case where the discharging flow rate is fixed at 500 µL/s and the aspiration flow rate is varied in the second step (a).
Fig. 9 is a graphical representation showing the relationship between the amount of nucleic acids contained in the mixed solution before aspiration and the amount of nucleic acids contained in the mixed solution after discharging, in the case where the discharging flow rate is fixed at 200 µL/s and the aspiration flow rate is varied in the second step (a).
Fig. 10 is a graphical representation showing the relationship between the amount of nucleic acids contained in the mixed solution before aspiration and the amount of nucleic acids contained in the mixed solution after discharging, in the case where the aspiration flow rate is fixed at 500 µL/s and the discharging flow rate is varied in the second step (a).

### Detailed Description of the Invention

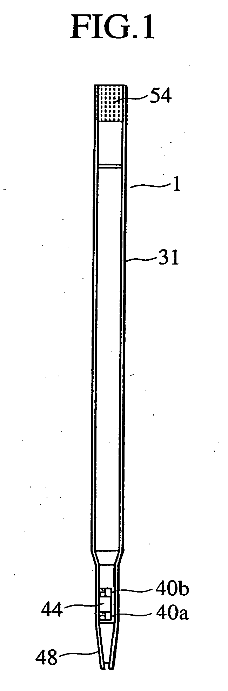

A detailed description is given below of the present invention. The description covers an apparatus and method for recovery of nucleic acids, a method for adjusting the apparatus for recovery of nucleic acids, an apparatus and method for producing nucleic acids, and a method for examining the concentration of nucleic acids. The following description refers to a nucleic acid-purifying apparatus which is equipped with a nucleic acid-capturing container such as a tip 31 as shown in Fig. 1. The tip 31 holds therein a solid phase 44, which captures nucleic acids and, upon cleaning with a cleaning solution, releases the captured nucleic acids subsequently. This apparatus is exemplary only and it not intended to restrict the scope of the present invention.

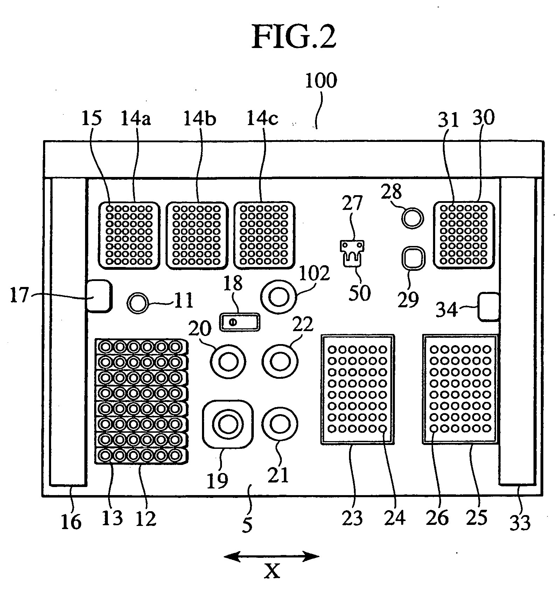

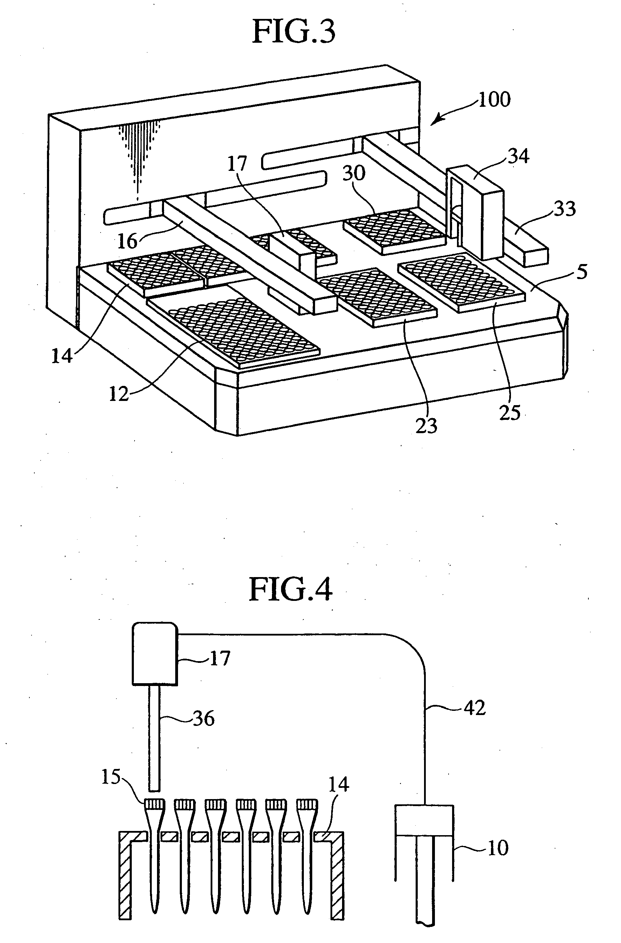

The nucleic acid-purifying apparatus shown in Figs. 2 and 3 includes two arms 16 and 33 (which are movable in the directions of arrows X shown in Fig. 2) and a work bench 5 which supports a sample rack 12 and a tip rack 30. The arms 16 and 33 have drive control means (not shown) so that they are moved horizontally parallel to the work bench 5. Mounted on the arms 16 and 33 are the nozzle holders 17 and 34, which have drive control means (not shown) so that they are moved in the lengthwise direction of the arms 16 and 33 and also moved relative to the work bench 5.

The arm 16 is provided with the nozzle holder 17 which holds the dispensing nozzle 36, as shown in Fig. 4. The dispensing nozzle 36 has a tip to which the dispensing tip 15 fits. The dispensing nozzle 36 is connected to a syringe pump 10 through a flexible tube 42. The syringe pump 36 is connected to a pure water supply system (not shown). The syringe pump 10 and the tube 42 are filled with pure water supplied from the pure water supply system.

The arm 33 is provided with the nozzle holder 34 to support the movable nozzle 39 through which solutions are aspirated and discharged. The movable nozzle 39 has a tip to which the nucleic acid-capturing tip 31 fits. The movable nozzle 39 is connected to a syringe pump 32 through a flexible tube 35. The syringe pump 32 is connected to a pure water supply system (not shown). The syringe pump 32 and the tube 35 are filled with pure water supplied from the pure water supply system.

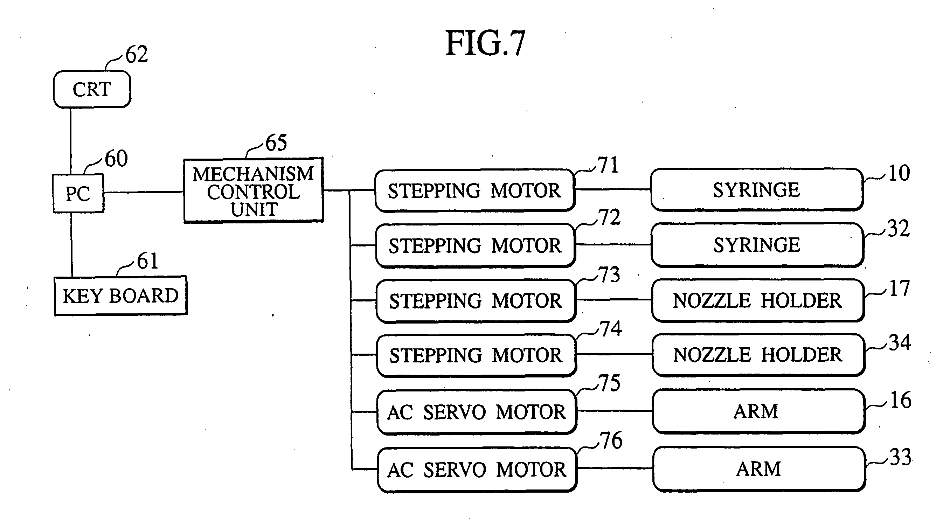

The syringe pump 32 has control means to control the pressure to be applied to the movable nozzle 39. The control means includes a stepping motor 73, a control mechanism 65, a PC 60, a keyboard 61, and a CRT 62, as shown in Fig. 7. The nucleic acid-capturing tip 31 attached to the movable nozzle 39 aspirates and discharges the mixed solution at a prescribed flow rate in response to the pressure applied to it by the syringe pump 32.

Incidentally, the arms 16 and 33 are mounted at different heights so that they do not collide with each other when they move horizontally parallel to the work bench 5.

On the work bench 5 are arranged a sample rack 12 to hold sample containers 13, a container rack 23 to hold working containers 24 for processing, a container storing rack 25 to hold containers 26 for purified products, tip racks 14a, 14b, and 14c to hold dispensing tips 15, and a tip rack 30 to hold nucleic acid-capturing tips 31. Moreover, on the work bench 5 are arranged the first to fifth reagent bottles 21, 22, 19, 102, and 20, which are used respectively in the first to fifth steps mentioned later. In addition, the work bench 5 has three ports for discharging. The first port is a solution receiver 11 to receive pure water discharged from the dispensing nozzle 36 at the time of priming. The first port is also the home position of the dispensing nozzle 36. The second port is a solution receiver 28 to receive pure water discharged from the movable nozzle 39 at the time of priming. The second port is also the home position of the movable nozzle 39. The third port is a waste receiver 29 into which unnecessary mixed solution is discharged from the nucleic acid-capturing tip 31. The work bench 5 is also provided with a tip remover 27 which removes the dispensing tip 15 and the nucleic acid-capturing tip 31. Incidentally, under the tip remover 27 is a waste tip receiver 50.

The sample rack 12 holds forty-eight sample containers 13 arranged in eight rows and six columns, for example. The sample container 13 holds a nucleic acid-containing sample, which includes vital samples (such as whole blood, serum, sputum, and urine), biological samples (such as cultured cells and bacteria), nucleic acid held in gel after electrophoresis, reaction products of DNA amplifying enzyme, and any substance containing crude nucleic acids. Incidentally, "nucleic acid" embraces DNA and RNA of two-chain structure or one-chain structure (throughout or partially).

The container rack 23 has a temperature control mechanism to keep the working containers 24 held therein at a prescribed temperature. The temperature control mechanism keeps at a prescribed temperature the working containers 24 held in the container rack 23 and the mixed solutions held in the working containers 24.

The container storing rack 25 holds forty-eight purified product containers 13 arranged in eight rows and six columns, for example. The purified product container 26 holds a purified solution obtained by purifying nucleic acid components from a nucleic acid-containing sample.

The tip racks 14a, 14b, and 14c each have openings to hold dispensing tips 15 therein, as shown in Fig. 4. They take on a boxy shape high enough to house the dispensing tips 15 without their ends coming into contact with the workbench 5. In other words, the dispensing tips 15 in their resting state are inserted into the openings of the tip racks 14a, 14b, and 14c. The dispensing tip 15 is attached to the end of the dispensing nozzle 36 by press fitting.

The tip rack 30 holds forty-eight nucleic acid-capturing tips 31 (shown in Fig. 1) arranged in eight rows and six columns, for example. Fig. 1 illustrates an example of the nucleic acid-capturing tip 31, which is formed such that the inside diameter gradually decreases in going downward from the head 54 to the lower end 48. The nucleic acid-capturing tip 31 is not specifically restricted in configuration so long as it includes a solid phase 44 (which captures nucleic acids), a space to hold the mixed solution containing nucleic acids, an opening through which the mixed solution containing nucleic acids is aspirated in and discharged from the space, and another opening that permits the internal pressure of the space to change.

The head 54 of the nucleic acid-capturing tip 31, which functions as an opening for pressure adjustment, has an inside diameter (say, about 5.5 mm) that permits airtight fitting or press fitting into the end of the dispensing nozzle 36. The space to hold the solid phase 44 therein has an inside diameter of, say, about 3.0 mm. The end 48 (or the opening through which the solution is aspirated and discharged) has an inside diameter of, say, about 0.8 mm. The nucleic acid-capturing tip 31 is formed by injection molding from a transparent or translucent synthetic resin such as polypropylene.

The nucleic acid-capturing tip 31 is provided with two discoid stopping members 40a and 40b, which are about 3 mm apart. In the space between the stopping members 40a and 40b is held the solid phase 44. The stopping members 40a and 40b have a large number of pores which are large enough to permit easy passage of liquid and gas but small enough to prevent the solid phase 44 from escaping. In other words, the solid phase 44 is confined in the space held between the stopping members 40a and 40b, so that it does not flow out. The stopping members 40a and 40b are a cylindrical body, about 3.1 mm in diameter and about 2.0 mm high, formed by sintering from quartz particles, about 0.1 mm in diameter. They may also be formed from polyvinylidene fluoride or the like, which is hydrophilic and less liable to non-specific adsorption for nucleic acid components. Such a hydrophilic material improves purification and yields of nucleic acids because of its low non-specific adsorption for proteins and nucleic acids.

The solid phase 44 may be formed from quartz wool (from Toshiba Chemicals, Grade B, 6-12 µm, 5 mg) or flint glass powder (from Wako Pure Chemical Industries, LTD., Kogyo), or any other materials which are not specifically restricted. In this embodiment, quartz wool is a desirable material. Also, flint glass permits purification of nucleic acids in high yields because of its high content of silica capable of capturing nucleic acids. Other materials for the solid phase 44 include glass particles, silica particles, quartz filter paper (or crushed product thereof), and diatomaceous earth, all of which contain silicon oxide.

The first reagent bottle 21 holds a reagent to promote the release of nucleic acids from nucleic acid-containing samples. This reagent may be a buffer solution of MES containing 2% Triton X-100 and 5.5 mmol/L GTC. Note: Triton X-100 is a product of LKB.

MES stands for 2-morpholinoethanesulfonic acid, a product of Dojindo Laboratories, Kenkyusho.

GTC stands for guanidine thiocyanate, biochemical grade, from Wako Pure Chemical Industries, LTD., Kogyo. This reagent should preferably have a low viscosity. In addition, in the case where RNA as nucleic acid is to be recovered, the reagent should preferably be incorporated with an RNase inhibitor or guanidine thiocyanate for protection of RNA from decomposition by RNase.

The second reagent bottle 22 holds a reagent containing a substance which promotes the binding of nucleic acids to the solid phase 44. This reagent is one which is generally called chaotropic reagent. In the case where the nucleic acid to be recovered is RNA, it should preferably be incorporated with guanidine thiocyanate as in the case of the reagent held in the first reagent bottle 21. The final concentration should be higher than 3 mol/L and the final pH value should be that of acid.

The third reagent bottle 19 holds a reagent which washes out the substance contained in the reagent supplied from the second reagent bottle 22 (said substance promoting the binding of nucleic acids to the solid phase 44) while maintaining the binding of the nucleic acids to the solid phase 44. This reagent includes, for example, solutions containing more than 70% ethyl alcohol, isopropyl alcohol, or the like. These alcohols should preferably be used in low concentrations just enough for their washing action because they have an adverse effect on PCR for recovered nucleic acids. For this reason, 50% ethyl alcohol containing 25 mmol/L potassium acetate is desirable.

The fourth reagent bottle 102 holds a reagent which washes out ethyl alcohol (which is contained in the reagent held in the third reagent bottle 22) and prevents it from being carried to the subsequent step, while maintaining the binding of the nucleic acids to the solid phase 44. This reagent should preferably be a 50 mmol/L solution of potassium acetate, and the solution temperature of this reagent should preferably be lower than 20°C.

The fifth reagent bottle 20 holds a mixed solution containing a substance which elutes the nucleic acids from the solid phase 44. An example of the mixed solution is water of low salt content or pure water which contains 10 mmol/L Bicine (pH = 8.5) and 0.1 mmol/L EDTA.

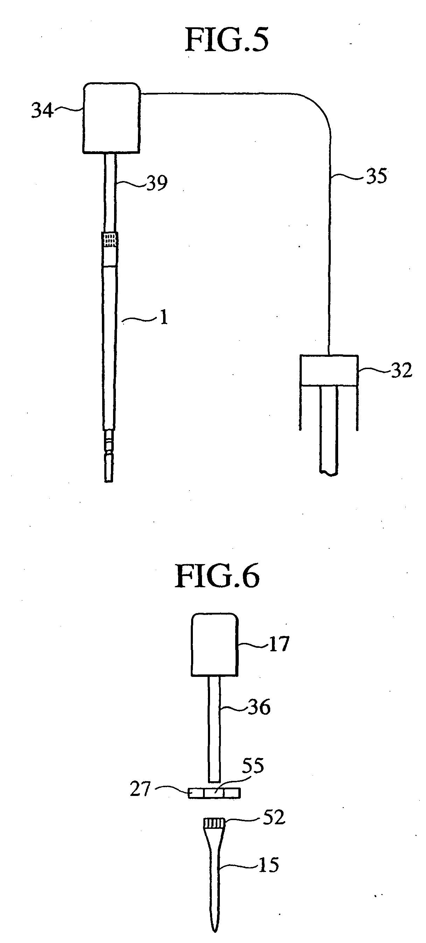

The tip remover 27 is a plate member having a slit 55 at a prescribed height from the top board of the work bench 5, as shown in Fig. 6. The slit 55 is narrower than the outside diameter of the head 52 of the dispensing tip 15 and the outside diameter of the head 54 of the nucleic acid-capturing tip 31 and is wider than the outside diameter of the dispensing nozzle 36. The tip remover 27 is manipulated in the following manner to remove the dispensing tip 15 from the dispensing nozzle 36. First, the arm 16 and the nozzle holder 17 are moved so that the dispensing nozzle 36 enters the slit 55 which is lower than the head 52. Then, the nozzle holder 17 is raised so that the head 52 comes into contact with the lower surface of the plate member, and the nozzle holder 17 is raised further so that the tip 15 drops off from the dispensing nozzle 36. The same procedure as above is carried out to remove the nucleic acid-capturing tip 31 from the movable nozzle 39 for solution aspiration and discharging.

The nucleic acid purifying apparatus 100 has an electric system as shown in Fig. 7. The entire system is controlled by a personal computer (PC) 60, which is connected to a keyboard 61, a CRT 62, and a mechanism control unit 65. The keyboard 61 is an interface through which to enter the condition of operation and the information of samples. The CRT 62 displays input information and warning information. The mechanism control unit 65 controls the stepping motor 71 (which drives the piston of the syringe pump 10 for aspiration and discharging), the stepping motor 72 (which drives the piston of the syringe pump 32 for aspiration and discharging), the stepping motor 73 (which moves the nozzle holder 17 in the vertical and horizontal directions), the stepping motor 74 (which moves the nozzle holder 34 in the vertical and horizontal directions), the AC servo motor 75 (which moves the arm 16 in the horizontal direction), and the AC servo motor 76 (which moves the arm 33 in the horizontal direction). These stepping motors and servo motors are driven according to the program available from the PC 60. The program may be entered through the keyboard 61 or selected from previously installed programs in the PC 60.

The nucleic acid purifying apparatus 100 constructed as mentioned above is used to recover (or produce) nucleic acids from a nucleic acid-containing sample through the first to fifth steps explained in the following. The nucleic acid-containing sample used for demonstration is a serum of a patient suffering from hepatitis type C (conforming to WHO International Standard) which is diluted with a serum negative for hepatitis type C at a tenfold step so that the resulting concentration ranges from 10⁶ IU/mL to 10² IU/mL.

The first step is designed to liberate nucleic acids from a nucleic acid-containing sample. In the first step, the arm 16 and the nozzle holder 17 are moved, and the dispensing tip 15 is attached to the end of the dispensing nozzle 36. The syringe pump 10 is actuated so as to aspirate 200 µL of nucleic acid-containing sample from the sample container 13 into the dispensing tip 15 and then discharge it into the working container 24. The arm 16 and the nozzle holder 17 are moved, and the dispensing tip 15 is removed by using the tip remover 27. (This procedure has been explained above with reference to Fig. 6.) The arm 16 and the nozzle holder 17 are moved, and the dispensing tip 15 (not yet used) is attached to the end of the dispensing nozzle 36. The syringe pump 10 is actuated so as to aspirate 700 µL of the first reagent from the first reagent bottle 21 into the dispensing tip 15 and then discharge it into the working container 24 (which has already received the nucleic acid-containing sample). The syringe pump 10 is actuated five times so as to aspirate and discharge the mixed solution from and into the working container 24 through the dispensing tip 15. This operation ensures thorough mixing. The mixed solution is allowed to stand for 10 minutes. During this period, the arm 16 and the nozzle holder 17 are moved, and the dispensing tip 15 (which has been used) is removed by using the tip remover 27.

The second step (a) and the second step (b) are designed to bring the mixed solution containing liberated nucleic acids into contact with the solid phase 44, thereby causing the solid phase 44 to adsorb (or capture) the nucleic acids. In the second step (a), the arm 16 and the nozzle holder 17 are moved, and the dispensing tip 15 (not yet used) is attached to the end of the dispensing nozzle 36. The syringe pump 10 is actuated so as to aspirate 100 µL of the second reagent from the second reagent bottle 22 into the dispensing tip 15 and then discharge it into the working container 24 (which has already received the nucleic acid-containing sample and the first reagent). The syringe pump 10 is actuated five times so as to aspirate and discharge the mixed solution from and into the working container 24 through the dispensing tip 15. This operation ensures thorough mixing. The arm 33 and the nozzle holder 34 are moved, and the nucleic acid-capturing tip 31 is attached to the end of the movable nozzle 39 for solution aspiration and discharging. The arm 33 and the nozzle holder 34 are moved so that the nozzle holder 34 is positioned above the working container 24 which holds the nucleic acid-containing sample and the mixture of the first and second reagents. The syringe pump 32 is actuated so as to aspirate all the mixed solution into the nucleic acid-capturing tip 31.

The mixed solution which has been aspirated is then discharged into the working container 24 to such an extent that the upper level of the mixed solution does not go beyond the upper stopping member 40b of the nucleic acid-capturing tip 31. The mixed solution in the working container 24 is aspirated to such an extent that the boundary between the mixed solution and the air does not go beyond the lower stopping member 40a of the nucleic acid-capturing tip 31. This aspiration and discharging operation keeps the solid phase 44 filled with liquid at all times, while excluding air therefrom. After the aspiration and discharging operations are repeated ten times, the mixes solution in the working container 24 is entirely aspirated into the nucleic acid-capturing tip 31, and then air is aspirated to such an extent that the boundary between the mixed solution and the air does not go beyond the stopping member 40a. With the nucleic acid-capturing tip 31 containing the mixed solution, the arm 33 and the nozzle holder 34 are driven so that the nucleic acid-capturing tip 31 is moved to the waste receiver 29. The syringe pump 32 is driven so that the mixed solution which has been aspirated is discharged to such an extent that it does not go beyond the stopping member 40b. The apparatus stands ready for the ensuing step.

In the second step (b), the arm 16 and the nozzle holder 17 are moved, and the dispensing tip 15 (not yet used) is attached to the dispensing nozzle 36. The syringe pump 10 is actuated so as to aspirate 800 µL of the second reagent from the second reagent bottle 22 into the dispensing tip 15 and then discharge it into the working container 24 (which has already received the mixed solution). The arm 16 and the nozzle holder 17 are driven, and the dispensing tip 15 (which has been used) is removed by using the tip remover 27. At the same time, the arm 33 and the nozzle holder 34 are driven so that the nucleic acid-capturing tip 31 is moved to the position above the working container 24 (which has already received the second reagent). The syringe pump 32 is actuated to repeat the aspiration and discharging of the mixed solution five times in the same way as mentioned above. The mixed solution in the working container 24 is entirely aspirated and then air is aspirated to such an extent that the boundary between the mixed solution and the air does not go beyond the stopping member 40a. The arm 33 and the nozzle holder 34 are driven so that the nucleic acid-capturing tip 31 is moved to the position above the waste receiver 29. The syringe pump 32 is actuated so that the second reagent, which has been aspirated, is discharged to such an extent that its level does not go beyond the stopping member 40b. The apparatus stands ready for the ensuing step. During this period, the arm 16 and the nozzle holder 17 are driven, and the dispensing tip 15 (not yet used) is attached to the dispensing nozzle 36.

The third step (a) and the third step (b) are the washing steps to remove the second reagent and any components (other than nucleic acids) contained in the nucleic acid-containing sample from the nucleic acid-capturing tip 31 and the solid phase 44. In the third step (a), the syringe pump 10 is actuated so as to aspirate 400 µL of the third reagent from the third reagent bottle 19 into the dispensing nozzle 15 and then discharge it entirely into the working container 24. The arm 16 and the nozzle holder 17 are driven, so that the dispensing tip 15 (which has been used) is removed by using the tip remover 27. During this period, the arm 33 and the nozzle holder 34 are driven, so that the nucleic acid-capturing tip 31 is moved to the position above the working container 24 which has already received the third reagent. The syringe pump 32 is actuated to repeat the aspiration and discharging of the third reagent three times in the same way as the mixed solution mentioned above. In this way the first washing is performed on the nucleic acid-capturing tip 31 and the solid phase 44. The mixed solution in the working container 24 is entirely aspirated and then air is aspirated to such an extent that the boundary between the mixed solution and the air does not go beyond the stopping member 40a. The arm 33 and the nozzle holder 34 are driven, so that the nucleic acid-capturing tip 31 is moved to the position above the waste receiver 29. The syringe pump 32 is driven so as to discharge entirely the third reagent which has been aspirated. The apparatus stands ready for the ensuing step. During this period, the arm 16 and the nozzle holder 17 are driven, so that the dispensing tip 15 (not yet used) is attached to the dispensing nozzle 36. The syringe pump 10 is actuated so as to aspirate 800 µL of the third reagent from the third reagent bottle 19 and then discharge it entirely into the waste container 24.

In the third step (b), the arm 16 and the nozzle holder 17 are driven, so that the dispensing tip 15 (which has been used) is removed by using the tip remover 27. During this period, the arm 33 and the nozzle holder 34 are driven, so that the nucleic acid-capturing tip 31 is moved to the position above the working container 24 which has received the third reagent. The aspiration and discharging of the third reagent is repeated three times in the same way as the mixed solution mentioned above. In this way the second washing is performed on the nucleic acid-capturing tip 31 and the solid phase 44. The third reagent is discharged into the waste receiver 29 as in the case of the first washing. In particular, in the second washing, the syringe pump 32 is actuated when the nucleic acid-capturing tip 31 is at the waste receiver 29, so as to aspirate and discharge 1 mL of air into and from the nucleic acid-capturing tip 31 ten times. The apparatus stands ready for the ensuing step. In the third step (b), the operation for the third reagent may be repeated. Incidentally, this washing operation may be repeated further if necessary.

The fourth step is intended to wash out any component (such as ethyl alcohol) contained in the third reagent from the nucleic acid-capturing tip 31 and the solid phase 44. In the fourth step, the arm 16 and the nozzle holder 17 are driven, so that the dispensing tip 15 (not yet used) is attached to the dispensing nozzle 36. The syringe pump 10 is actuated so as to aspirate 200 µL of the fourth reagent from the fourth reagent bottle 102 into the dispensing tip 15 and to discharge it entirely into the waste container 24. The arm 16 and the nozzle holder 17 are driven, so that the dispensing tip 15 (which has been used) is removed by using the tip remover 27. During this period, the arm 33 and the nozzle holed 34 are driven, so that the nucleic acid-capturing tip 31 is moved to the position above the working container 24. The syringe pump 32 is actuated so as to repeat the aspiration and discharging of the fourth reagent three times. In this way, washing is performed on the nucleic acid-capturing tip 31 and the solid phase 44. The syringe pump 32 is actuated so as to aspirate entirely the fourth reagent from the working container 24 and to aspirate air. The arm 33 and the nozzle holder 34 are driven, so that the nucleic acid-capturing tip 31 is moved to the waste receiver 29. The syringe pump 32 is actuated so as to discharge the fourth reagent entirely from the nucleic acid-capturing tip 31. The aspiration and discharging of 1 mL of air into and from the nucleic acid-capturing tip 31 is repeated ten times.

The fifth step is intended to elute, for recovery, the nucleic acids which have adsorbed to the solid phase 44. In the fifth step, the arm 16 and the nozzle holder 17 are driven, so that the dispensing tip 15 (not yet used) is attached to the dispensing nozzle 36. The syringe pump 10 is actuated so as to aspirate 100 µL of the fifth reagent from the fifth reagent bottle 20 and discharge it entirely into the working container 24. The arm 16 and the nozzle holder 17 are driven, so that the dispensing tip 15 (which has been used) is removed by using the tip remover 27. During this period, the arm 33 and the nozzle holder 34 are driven, so that the nucleic acid-capturing tip 31 is moved to the position above the working containing 24 which has already received the fifth reagent. The syringe pump 32 is actuated so as to repeat the aspiration and discharging of the fifth reagent twenty times and finally discharge the fifth reagent entirely. In this way, the nucleic acids which have adsorbed to the solid phase 44 are eluted. The arm 33 and the nozzle holder 34 are driven, so that the nucleic acid-capturing tip 31 (which has been used) is removed by using the tip remover 27. During this period, the arm 16 and the nozzle holder 17 are driven so that the dispensing tip 15 (not yet used) is attached. The syringe pump 10 is actuated so as to aspirate the fifth reagent entirely from the working container 24. A small amount of air is aspirated. The arm 16 and the nozzle holder 17 are driven so that the dispensing tip 15 is moved to the position above the purified product container 26. The syringe pump 10 is actuated so as to discharge the fifth reagent entirely from the dispensing tip 15 into the purified product container 26. The arm 16 and the nozzle holder 17 are driven, so that the dispensing tip 15 (which has been used) is removed by using the tip remover 27.

The foregoing steps are all required to purify nucleic acids from the nucleic acid-containing sample held in the sample container 13. In the second step (a) and the second step (b), the nucleic acid-capturing tip 31 aspirates and discharges the mixed solution as the syringe pump 32 is actuated to apply a controlled pressure to the movable nozzle 39 for solution aspiration and discharging. In the nucleic acid purifying apparatus 100, the syringe pump 32 is controlled by a control means, so that it is possible to apply a desired pressure to the movable nozzle 39 for solution aspiration and discharging; as the result, it is possible to carry out the aspiration and discharging of the mixed solution at a desired rate.

The present inventors considered that it will be possible to certainly prevent the occurrence of bubbles near the solid phase 44 in the nucleic acid-capturing tip 31 by controlling the aspiration rate for each of the nucleic acid-capturing tips. They also considered that it will be possible to certainly discharge to the outside the bubbles which occur or exist near the solid phase 44 by controlling the discharging rate. And, as the result of verification experiments, they ascertained that it is possible to keep in a good state the contact between the mixed solution and the solid phase 44 by controlling the aspiration rate and the discharging rate, and consequently the solid phase can capture nucleic acids in high yields. In addition, they found that it is possible to control the recovery ratio of nucleic acids by controlling the aspiration rate and the discharging rate. Here, the recovery ratio of nucleic acids is defined as the ratio of Nr/Ns, where Ns is the initial concentration of nucleic acids in the mixed solution containing nucleic acids which is not yet aspirated into the nucleic acid-capturing container, and Nr is the concentration of recovered nucleic acids in the mixed solution which is discharged from the nucleic acid-capturing container after nucleic acids captured on the solid phase have been dissolved. Incidentally, the recovery ratio of nucleic acids should be calculated on the assumption that the volume of the mixed solution containing nucleic acids before aspiration into the nucleic acid-capturing container is equal to the volume of the mixed solution discharged from the nucleic acid capturing container after dissolution of nucleic acids captured on the solid phase. If the two volumes are not equal, it is necessary to multiply the concentration of nucleic acids by a certain factor so that the two volumes are regarded as equal. Also, the initial amount of nucleic acids can be calculated from the product of the initial concentration and the volume of the mixed solution containing nucleic acids before aspiration into the nucleic acid-capturing container. And, the amount of recovered nucleic acids can be calculated from the concentration of recovered nucleic acids and the amount of the mixed solution discharged from the nucleic acid-capturing container.

In what follows, the present invention will be described from the standpoint of the rate of recovery of nucleic acids. According to the present invention, it is desirable to control the aspiration and discharging rates such that the rate of recovery of nucleic acids is no less than 1% if the initial concentration is in the range of 10² IU/mL to 10⁶ IU/mL. (The initial concentration is the concentration of nucleic acids (such as genome nucleic acids of virus) contained in the mixed solution before aspiration.) This is because, if the rate of recovery of nucleic acids is no less than 1%, it is possible to examine (qualitatively) whether or not the mixed solution before aspiration contains desired nucleic acids. Incidentally, the rate of recovery of nucleic acids decreases as the initial concentration increases. Therefore, if the initial concentration is high (say, 10⁶ IU/mL), it is necessary to control the aspiration and discharging rates such that the rate of recovery of nucleic acid is no less than 1%. In this way it is possible to make corrections with certainty.

Also, in the case where the initial concentration is in the range of 10² IU/mL to 10⁶ IU/mL, it is desirable to control the aspiration and discharging rates such that the rate of recovery of nucleic acids is no less than 10%. Any apparatus used for nucleic acid analysis usually has one order of error allowance; therefore, if the rate of recovery of nucleic acids is no less than 10%, it is possible to carry out accurate quantitative analysis for nucleic acids contained in the mixed solution before aspiration. Incidentally, for the same reason as mentioned above, if the initial concentration is high, it is possible to carry out accurate quantitative analysis by controlling the aspiration and discharging rates such that the rate of recovery of nucleic acids is no less than 10%.

Moreover, in the case where the initial concentration is in the range of 10² IU/mL to 10⁶ IU/mL, it is desirable to control the aspiration and discharging rates such that the rate of recovery of nucleic acids is no less than 50%. The high rate of recovery of nucleic acids increases the efficiency of purification of nucleic acids (or the productivity of nucleic acids). Also, the rate of recovery of nucleic acids is constant regardless of the concentration of nucleic acids contained in the mixed solution before aspiration; therefore, it is possible to accurately grasp the concentration of the nucleic acids contained in the mixed solution before aspiration. Incidentally, for the same reason mentioned above, the same effect is produced by controlling the aspiration and discharging rates such that the rate of recovery of nucleic acids is no less than 50% if the initial concentration is high.

Moreover, in the case where the initial concentration (or the concentration of nucleic acids contained in the mixed solution before aspiration) is in the range of 10² IU/mL to 10⁶ IU/mL, it is desirable to control the aspiration and discharging rates such that the rate of recovery of nucleic acids is approximately constant regardless of the initial concentration. "Approximately constant" means the rate of recovery of nucleic acids fluctuates within a range of ±10% for the initial concentration In other words, in the case where the initial concentration is in the range of 10² IU/mL to 10⁶ IU/mL, the difference between the maximum rate of recovery of nucleic acids and the minimum rate of recovery of nucleic acids is within 20%. It follows, therefore, that when the present invention is used for an apparatus of determining the concentration of nucleic acids which calculates the initial concentration from the amount of nucleic acids contained in the mixed solution after discharging, then the apparatus can determine the initial concentration within an error of 10% (which is an allowance of error of the measuring apparatus used in the field of nucleic acid detection). Moreover, in the case where the present invention is used for an apparatus of determining nucleic acids in the medical scene, the apparatus can determine the concentration of specific nucleic acids contained in the patient's body fluid within an error of 10%.

It is more desirable that the fluctuation of the rate of recovery of nucleic acids is within a range of ±10% for the initial concentration. If the rate of recovery is approximately constant, it is possible to accurately determine the amount of nucleic acids contained in the mixed solution containing nucleic acids by determining the nucleic acids contained in the mixed solution which has been recovered into the purified product container 26.

The present invention will be described from the standpoint of the rate of aspiration and discharging. According to the present invention, it is desirable to control the rate of aspiration in such a way that the rate of aspiration for each nucleic acid-capturing tip is no higher than 500 µL/s, preferably no higher than 350 µL/s. In this way it is possible to prevent the occurrence of bubbles in the vicinity of the solid phase 44 in the nucleic acid-capturing tip 31.

It is desirable to control the rate of aspiration in such a way that the rate of discharging for each nucleic acid-capturing tip is no higher than 1000 µL/s, preferably no higher than 500 µL/s. In the case where the rate of aspiration for each nucleic acid-capturing tip is higher than 500 µL/s, the rate of discharging should be higher than this rate of aspiration. In this way, it is possible to certainly discharge to outside the bubbles which occur or exist in the vicinity of the solid phase 44.

To be concrete, in the case where the concentration of nucleic acids (e.g., HCV) contained in the mixed solution before aspiration is 102 IU/mL to 10⁶ IU/mL, it is possible to keep the fluctuation of the rate of recovery of nucleic acids within ±10% for the concentration of each nucleic acid (or the concentration of virus). In other words, the difference between the maximum rate of recovery of nucleic acids and the minimum rate of recovery of nucleic acids is within 20%.

Also, in the case where the rate of aspiration and the rate of discharging are adequately controlled, it is possible to keep the fluctuation of the rate of recovery of nucleic acids within ±5% for each concentration. In this way, it is possible to recover the nucleic acids contained in the mixed solution at an approximately constant rate of recovery regardless of the concentration of nucleic acids contained in the mixed solution before aspiration. It is understood from the foregoing that the present invention can be applied not only to the apparatus and method for purifying nucleic acids from the mixed solution containing nucleic acids but also to the apparatus for determining the initial concentration of the nucleic acids in the mixed solution before aspiration from the amount of recovered nucleic acids.

In addition, the present invention may be described as follows from another standpoint. In the case where the concentration of nucleic acids (e.g., HCV) contained in the mixed solution before aspiration is 10⁶ IU/mL, it is possible to keep the rate of recovery of nucleic acids higher than 1%. In this way it is possible to qualitatively judge the specific nucleic acids contained in the mixed solution before aspiration. In the case where the rate of aspiration and the rate of discharging are adequately controlled, it is possible to keep the rate of recovery of nucleic acids higher than 10% if the concentration of nucleic acids (e.g., HCV) contained in the mixed solution before aspiration is 10⁶ IU/mL. (This rate of recovery of nucleic acids is within the range of error allowance of the measuring apparatus used at present in the field of this technology.) As mentioned above, the present invention is of practical value. In particular, in the case where the rate of aspiration and the rate of discharging are adequately controlled, it is possible to keep the rate of recovery of nucleic acids higher than 50% if the concentration of nucleic acids contained in the mixed solution before aspiration is 10⁶ IU/mL. If the concentration of nucleic acids contained in the mixed solution before aspiration is as low as about 10² IU/mL, the rate of recovery of nucleic acids is often higher than 50% regardless of the rate of aspiration and discharging, and the rate of recovery of nucleic acids decreases with the increasing concentration of nucleic acids contained in the mixed solution before aspiration. Therefore, if it is possible to increase the rate of recovery of nucleic acids above 50% in the case where the concentration of nucleic acids contained in the mixed solution before aspiration is 10⁶ IU/mL, then it is possible to recover at an approximately constant rate the nucleic acids contained in the mixed solution.

The foregoing was verified in the following manner. The amount of nucleic acids contained in the mixed solution before aspiration is compared with the amount of nucleic acids contained in the mixed solution after the completion of steps when the rate of aspiration and discharging of the mixed solution in the second step (a) is changed. Incidentally, the mixed solution is a serum of a patient suffering from hepatitis type C (conforming to WHO International Standard) which is diluted with a serum negative for hepatitis type C at a tenfold step so that the resulting concentration ranges from 10⁶ IU/mL to 10² IU/mL. The initial concentration of the mixed solution is in the range of 10⁶ IU/mL to 10² IU/mL in view of the fact that the amount of virus present in the serum of hepatitis type C is lower than 10⁷ IU/mL and the goal for therapy by administration of interferon is regarded as 10⁵ IU/mL.

Incidentally, the amount of nucleic acids contained in the mixed solution after the completion of steps was evaluated by using the PCR apparatus (COBAS-AMPLICOR from of Roche) and HCV v2.0 reagent. To be concrete, a mixture is prepared from 48.5 µL of the mixed solution to be evaluated, 41.7 µL of HCV master mix v2.0, 8.3 µL of HCV manganese reagent, and 1.5 µL of HCV internal control v2.0. The apparatus is operated according to the manual attached to the reagent kit. The mixed solution which has undergone all the steps is diluted stepwise, and the thus obtained samples were analyzed for the amount of nucleic acids contained therein.

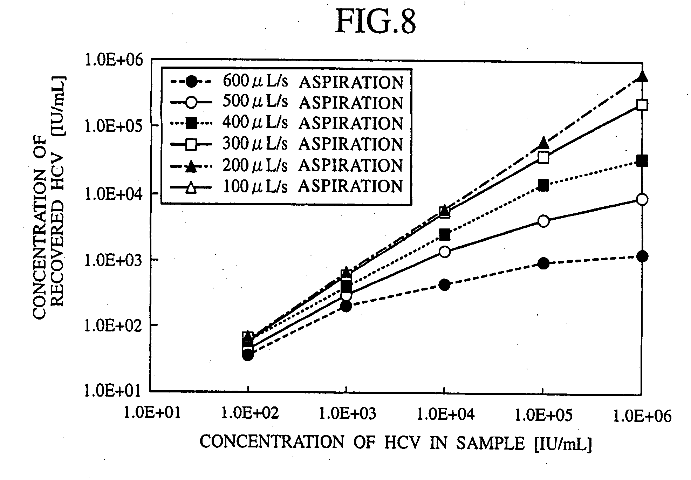

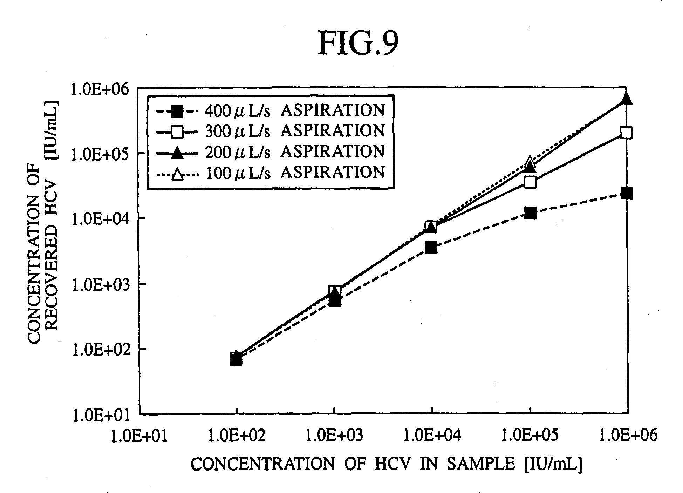

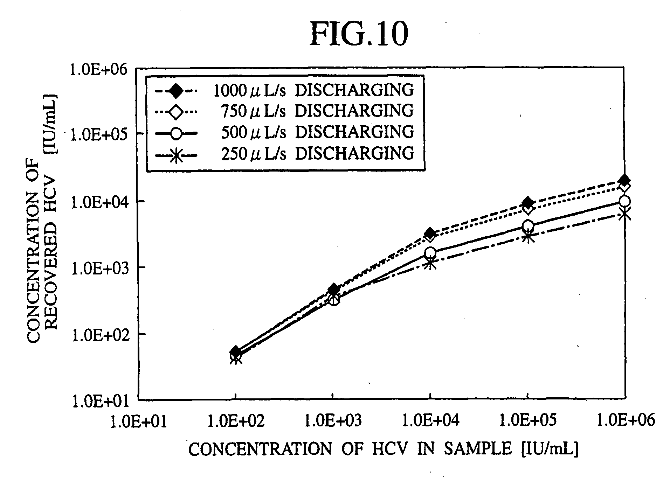

The result of analysis is shown in Figs. 8 to 10. The result shown in Fig. 8 was obtained when the rate of discharging was fixed at 500 µL/s and the rate of aspiration was varied in the second step (a). The result shown in Fig. 9 was obtained when the rate of discharging was fixed at 200 µL/s and the rate of aspiration was varied in the second step (a). The result shown in Fig. 10 was obtained when the rate of aspiration was fixed at 500 µL/s and the rate of discharging was varied in the second step (a). In Figs. 8 to 10, the abscissa represents the concentration of nucleic acids contained in the mixed solution and the ordinate represents the concentration of nucleic acids contained in the mixed solution after the completion of steps.

The results shown in Figs. 8 to 10 prove that it is possible to keep the rate of recovery of nucleic acids higher than 1% by adequately controlling the rate of aspiration and discharging if the initial concentration of the mixed solution before aspiration is in the range of 10² IU/mL to 10⁶ IU/mL. In Fig. 8, the adequate rate of aspiration ranges from 100 to 400 µL/s. In Fig. 9, the adequate rate of aspiration ranges from 100 to 400 µL/s. In Fig. 10, the adequate rate of aspiration ranges from 100 to 400 µL/s. It was also confirmed that it is possible to keep the rate of recovery of nucleic acids higher than 1% by controlling the rate of aspiration such that the rate of aspiration for each nucleic acid-capturing tip is lower than 500 µL/s if the initial concentration of the mixed solution before aspiration is 10⁶ IU/mL.

The results shown in Figs. 8 and 9 prove that it is possible to keep the rate of recovery of nucleic acids higher than 10% by adequately controlling the rate of aspiration and discharging if the initial concentration of the mixed solution before aspiration is in the range of 10² IU/mL to 10⁶ IU/mL. In Fig. 8, the adequate rate of aspiration ranges from 100 to 300 µL/s. In Fig. 9, the adequate rate of aspiration ranges from 100 to 400 µL/s. It was also confirmed that it is possible to keep the rate of recovery of nucleic acids higher than 10% by controlling the rate of aspiration such that the rate of aspiration for each nucleic acid-capturing tip is lower than 350 µL/s if the initial concentration of the mixed solution before aspiration is 10⁶ IU/mL.

The results shown in Figs. 8 and 9 prove that it is possible to keep the rate of recovery of nucleic acids higher than 50% by adequately controlling the rate of aspiration and discharging if the initial concentration of the mixed solution before aspiration is in the range of 10² IU/mL to 10⁶ IU/mL. In Fig. 8, the adequate rate of aspiration ranges from 100 to 200 µL/s. In Fig. 9, the adequate rate of aspiration ranges from 100 to 200 µL/s.

It was also confirmed from the result shown in Fig. 10 (in which the adequate rate of aspiration ranges from 100 to 400 µL/s) that it is possible to keep the rate of recovery of nucleic acids higher than 1%, if the initial concentration of the mixed solution before aspiration is 10⁶ IU/mL, by making the rate of discharging larger than the rate of aspiration when the rate of aspiration for each nucleic acid-capturing tip is greater than 500 µL/s.

By controlling the rate of aspiration and discharging as mentioned above, it is possible to recover nucleic acids differing in concentrations from a sample of the same concentration with the help of the same apparatus and reagent in combination. This suggests that there is the possibility that the analysis gives a lower virus concentration than an actual value if the rate of aspiration and discharging is selected incorrectly and that the apparatus is extremely poor in reliability if the rate of aspiration and discharging is not controlled adequately. Therefore, it is of crucial importance that the rate of aspiration and discharging should be adequately controlled under prescribed conditions.

If the rate of aspiration and the rate of discharging are combined in an adequate range so that good reproducibility is obtained, it is possible to calculate the concentration before recovery from the concentration of recovered nucleic acids by calibration even though the rate of recovery is low.

"Calibration" means here the same procedure employed for the ordinary measuring apparatus. This procedure consists of preparing a calibration curve from the analysis of known samples which is carried out in the same way as for unknown samples. The correct concentrations of unknown samples can be calculated by using this calibration curve. In other words, a relationship is previously established among the concentration of nucleic acids contained in the mixed solution, the concentration or amount of nucleic acids that can be recovered, and the rate of aspiration and discharging. By comparing this relationship with the rate of aspiration and discharging, it is possible to calculate the concentration of nucleic acids contained in the sample.

In addition, if this information is used to adequately control the rate of aspiration and the rate of discharging, it is possible to reduce time for processing. For example, in Fig. 8, in order to accomplish recovery at a constant rate of recovery from low concentrations to high concentrations, it is necessary to keep the rate of aspiration lower than 200 µL/s, but by performing calibration, it is possible to make the rate of aspiration at 500 µL/s, and when the same amount of solution is aspirated, it is possible to reduce the necessary time to 2/5, and it is a very effective technique to reduce time for processing. The method like this is not suitable when it is desirable to obtain a large amount of nucleic acids from a certain sample; but in the case where it is desirable to determine the concentration in a sample as in clinical examination, it can be said to be a very effective technique.

Also, this information may be used to adjust the means to control the rate of aspiration and the rate of discharging of the nucleic acid recovering apparatus in relation to the characteristics of the sample and the applications of the apparatus and to control the rate of aspiration and the rate of discharging, thereby changing the rate of recovery of nucleic acids and the time required for processing. In this way the apparatus for recovery of nucleic acids finds wider areas of application and it becomes possible to use an apparatus more suitable for the sample and use.

As mentioned above, the principle of the present invention can be applied to a method for accurately determining the concentration of nucleic acids in a mixed solution containing nucleic acids and to a method for efficiently producing a nucleic acid-containing solution from a mixed solution containing nucleic acids.

### Industrial Applicability

According to the present invention, it is possible to control the state in which the solution containing nucleic acids comes into contact with the solid phase in the nucleic acid-capturing container. According to the present invention, it is possible to provide an apparatus and method for efficiently recovering nucleic acids contained in said solution, a method for adjusting the apparatus for recovering nucleic acids, an apparatus and method for producing nucleic acids, and a method for determining the concentration of nucleic acids.

## Claims

1. A nucleic acid recovering method for recovering nucleic acids from a first solution by the steps of
(i) aspirating the first solution into a nucleic acid capturing container (31) having a solid phase (44) capable of capturing nucleic acids, and discharging the solution, thereby causing said solid phase (44) to capture nucleic acids, and then
(ii) aspirating a second solution into said container (31), said second solution liberating the captured nucleic acids from said solid phase (44), thereby recovering said captured nucleic acids,
wherein the method further comprises a calibration by establishing a relationship between the rate of recovery Nr/Ns and rates of aspiration and discharging for step (i), where Ns is the concentration of nucleic acids in said first solution, and Nr is the concentration of recovered nucleic acids, and
wherein the rate of aspiration and the rate of discharging in step (i) are controlled using the established relationship so that the ratio of Nr/Ns is no smaller than 10%..

2. The method of claim 1, wherein said rate of aspiration and said rate of discharging are controlled so that the ratio of Nr/Ns is no smaller than 50%.

3. The method of claim 1 or 2, comprising the following steps:
(1) liberating nucleic acids from a sample, thereby preparing a solution containing the liberated nucleic acids,
(2) capturing nucleic acids by adding to the solution containing the liberated nucleic acids a substance which promotes the binding between said nucleic acids and the solid phase (44) thereby obtaining said first solution, and performing said step (i),
(3) washing by aspirating into said container (31) a third solution capable of removing components other than the nucleic acids captured on said solid phase (44) and then discharging the third solution from said container (31), and
(4) eluting the captured nucleic acids from said solid phase (44), by performing said step (ii) and eluting said second solution from said container (31),
wherein the rate of discharge in step (i) is no larger than 1000 µL/s, excluding 0 µL/s.

4. The method of any of claims 1 to 3, wherein said rate of aspiration and/or said rate of discharging are controlled so that the rate of recovery of nucleic acids is approximately constant when the concentration of nucleic acids in the first solution is in the range of 10² IU/mL to 10⁶ IU/mL.

5. The method of any of claims 1 to 4, further comprising a step of calculating the concentration of nucleic acids in the first solution from the concentration of said liberated nucleic acids on the basis of said relationship among the rate of recovery and the rates of aspiration and discharging.

6. The method of any of claims 1 to 5, wherein the rate of aspiration is no larger than 500 µL/s, preferably no larger than 350 µL/s, each excluding 0 µL/s.

7. The method of any of claims 1 to 6, wherein the rate of discharging is no larger than 1000 µL/s, preferably no larger than 500 µL/s, each excluding 0 µL/s.

8. The method of any of claims 1 to 5, wherein the rate of aspiration is no smaller than 500 µL/s and the rate of discharging is greater than the rate of aspiration.

9. The method of any of claims 1 to 8, wherein the rate of aspiration is controlled in step (i) to be higher than necessary, in said relationship, to accomplish a constant rate of recovery from low to high concentrations of nucleic acids, and the method comprises a step of calculating the concentration of nucleic acids in the first solution from the concentration of said liberated nucleic acids and said relationship among the rate of recovery and the rates of aspiration and discharging.

## Patentansprüche

1. Nukleinsäure-Gewinnungsverfahren zum Gewinnen von Nukleinsäuren aus einer ersten Lösung, mit folgenden Schritten:
(i) Ansaugen der ersten Lösung in einen Nukleinsäure-Aufnahmebehälter (31) mit einer festen Phase (44), die Nukleinsäuren aufnehmen kann, und Abgeben der Lösung, wobei bewirkt wird, dass die feste Phase (44) Nukleinsäuren aufnimmt, und dann
(ii) Ansaugen einer zweiten Lösung in den Behälter (31), die die aufgenommenen Nukleinsäuren aus der festen Phase (44) freisetzt, wodurch die aufgenommenen Nukleinsäuren gewonnen werden,
wobei das Verfahren außerdem ein Kalibrieren durch Herstellen einer Beziehung zwischen der Gewinnungsrate Nr/Ns und den Ansaug- und Abgabegeschwindigkeiten für Schritt (i) umfasst, wobei Ns die Konzentration von Nukleinsäuren in der ersten Lösung und Nr die Konzentration gewonnener Nukleinsäuren ist, und
wobei die Ansauggeschwindigkeit und die Abgabegeschwindigkeit in Schritt (i) unter Verwendung der hergestellten Beziehung so gesteuert werden, dass das Verhältnis Nr/Ns nicht kleiner als 10% ist.

2. Verfahren nach Anspruch 1, wobei die Ansauggeschwindigkeit und die Abgabegeschwindigkeit so gesteuert werden, dass das Verhältnis Nr/Ns nicht kleiner als 50% ist.

3. Verfahren nach Anspruch 1 oder 2 mit folgenden Schritten:
(1) Freisetzen von Nukleinsäuren aus einer Probe unter Herstellung einer Lösung, die die freigesetzten Nukleinsäuren enthält,
(2) Aufnehmen der freigesetzten Nukleinsäuren, indem zu der die freigesetzten Nukleinsäuren enthaltenden Lösung ein Stoff hinzugefügt wird, der die Bindung zwischen den Nukleinsäuren und der festen Phase (44) fördert, wodurch die erste Lösung erhalten wird, und indem Schritt (i) ausgeführt wird,
(3) Waschen durch Ansaugen einer dritten Lösung in den Behälter (31), die andere Bestandteile als die von der festen Phase (44) aufgenommenen Nukleinsäuren entfernen kann, und dann Abgeben der dritten Lösung aus dem Behälter (31), und
(4) Eluieren der aufgenommenen Nukleinsäuren aus der festen Phase (44) durch Ausführen des Schritts (ii) und Eluieren der zweiten Lösung aus dem Behälter (31),
wobei die Abgabegeschwindigkeit in Schritt (i) nicht größer als 1000 µL/s unter Ausschluss von 0 µL/s ist.

4. Verfahren nach einem der Ansprüche 1 bis 3, wobei die Ansauggeschwindigkeit und/oder die Abgabegeschwindigkeit so gesteuert werden, dass die Gewinnungsrate der Nukleinsäuren etwa konstant ist, wenn die Konzentration an Nukleinsäuren in der ersten Lösung im Bereich von 10² IU/mL bis 10⁶ IU/mL liegt.

5. Verfahren nach einem der Ansprüche 1 bis 4, mit einem Schritt zum Berechnen der Konzentration von Nukleinsäuren in der ersten Lösung aus der Konzentration der freigesetzten Nukleinsäuren aufgrund der Beziehung zwischen der Gewinnungsrate und den Ansaug- und Abgabegeschwindigkeiten.

6. Verfahren nach einem der Ansprüche 1 bis 5, wobei die Ansauggeschwindigkeit nicht größer als 500 µL/s, vorzugsweise nicht größer als 350 µL/s, jeweils unter Ausschluss von 0 µL/s ist.

7. Verfahren nach einem der Ansprüche 1 bis 6, wobei die Abgabegeschwindigkeit nicht größer als 1000 µL/s, vorzugsweise nicht größer als 500 µL/s, jeweils unter Ausschluss von 0 µL/s ist.

8. Verfahren nach einem der Ansprüche 1 bis 5, wobei die Ansauggeschwindigkeit nicht kleiner als 500 µL/s und die Abgabegeschwindigkeit größer als die Ansauggeschwindigkeit ist.

9. Verfahren nach einem der Ansprüche 1 bis 8, wobei die Ansauggeschwindigkeit in Schritt (i) auf einen höheren Wert gesteuert wird als in der genannten Beziehung notwendig ist, um von niedrigen bis hohen Konzentrationen von Nukleinsäuren eine konstante Gewinnungsrate zu erzielen, und wobei das Verfahren einen Schritt zum Berechnen der Konzentration von Nukleinsäuren in der ersten Lösung aus der Konzentration der freigesetzten Nukleinsäuren und der genannten Beziehung zwischen der Gewinnungsrate und den Ansaug- und Abgabegeschwindigkeiten enthält.

## Revendications

1. Procédé de récupération d'acide nucléique pour récupérer des acides nucléiques dans une première solution par les étapes consistant à
(i) aspirer la première solution dans un récipient (31) de capture d'acide nucléique ayant une phase solide (44) capable de capturer les acides nucléiques, et déverser la solution, ce qui fait que ladite phase solide (44) capture les acides nucléiques, puis
(ii) aspirer une deuxième solution dans ledit récipient (31), ladite deuxième solution libérant les acides nucléiques capturés de ladite phase solide (44), pour ainsi récupérer lesdits acides nucléiques capturés,
le procédé comprenant en outre un étalonnage par établissement d'une relation entre le taux de récupération Nr/Ns et les taux d'aspiration et de déversement pour l'étape (i), Ns étant la concentration d'acides nucléiques dans ladite première solution, et Nr étant la concentration d'acides nucléiques récupérés, et
le taux d'aspiration et le taux de déversement de l'étape (i) étant contrôlés en utilisant la relation établie de sorte que le ratio Nr/Ns ne soit pas inférieur à 10 %.

2. Procédé selon la revendication 1, dans lequel ledit taux d'aspiration et ledit taux de déversement sont contrôlés de telle sorte que le ratio Nr/Ns n'est pas inférieur à 50 %.

3. Procédé selon la revendication 1 ou 2, comprenant les étapes suivantes consistant à :
(1) libérer les acides nucléiques d'un échantillon, pour préparer ainsi une solution contenant les acides nucléiques libérés,
(2) capturer les acides nucléiques en ajoutant à la solution contenant les acides nucléiques libérés une substance qui favorise la liaison entre lesdits acides nucléiques et la phase solide (44) pour obtenir ainsi ladite première solution, et réaliser ladite étape (i),
(3) laver par aspiration dans ledit récipient (31) une troisième solution capable d'éliminer les composants autres que les acides nucléiques capturés sur ladite phase solide (44) puis déverser la troisième solution depuis ledit récipient (31), et
(4) éluer les acides nucléiques capturés de ladite phase solide (44) en réalisant ladite étape (ii) et en éluant ladite deuxième solution dudit récipient (31),
le taux de déversement à l'étape (i) n'étant pas supérieur à 1 000 µl/s et excluant 0 µl/s.

4. Procédé selon l'une quelconque des revendications 1 à 3, dans lequel ledit taux d'aspiration et/ou ledit taux de déversement sont contrôlés de telle sorte que le taux de récupération des acides nucléiques soit approximativement constant lorsque la concentration d'acides nucléiques dans la première solution est dans la plage de 10² UI/ml à 10⁶ UI/ml.

5. Procédé selon l'une quelconque des revendications 1 à 4, comprenant en outre une étape consistant à calculer la concentration d'acides nucléiques dans la première solution à partir de la concentration desdits acides nucléiques libérés en se fondant sur ladite relation entre le taux de récupération et les taux d'aspiration et de déchargement.

6. Procédé selon l'une quelconque des revendications 1 à 5, dans lequel le taux d'aspiration n'est pas supérieur à 500 µl/s, de préférence pas supérieur à 350 µl/s, chacun excluant 0 µl/s.

7. Procédé selon l'une quelconque des revendications 1 à 6, dans lequel le taux de déversement n'est pas supérieur à 1 000 µl/s, de préférence pas supérieur à 500 µl/s, chacun excluant 0 µl/s.

8. Procédé selon l'une quelconque des revendications 1 à 5, dans lequel le taux d'aspiration n'est pas inférieur à 500 µl/s et le taux de déversement est supérieur au taux d'aspiration.

9. Procédé selon l'une quelconque des revendications 1 à 8, dans lequel le taux d'aspiration est contrôlé à l'étape (i) de manière à être supérieur à ce qui est nécessaire, dans ladite relation, pour accomplir un taux de récupération constant de concentrations faibles à élevées d'acides nucléiques libérés, et le procédé comprend une étape consistant à calculer la concentration d'acides nucléiques dans la première solution à partir de la concentration desdits acides nucléiques et de ladite relation entre le taux de récupération et les taux d'aspiration et de déversement.
